# EUROPEAN PATENT APPLICATION

(11) **EP 1 020 190 A2**
(43) Date of publication of application: **19.07.2000**
(21) Application number: 99308148.8
(22) Date of filing: 15.10.1999
(51) Int. Cl.: A61K 31/52, A61K 31/505, A61K 31/50, A61P 13/08, A61P 13/00

(54) **Treatment of BPH with cGMP elevators**

(30) Priority: 21.10.1998 US 105097 P
(71) Applicant: Pfizer Products Inc., Groton, Connecticut 06340 (US)
(72) Inventor: Wyllie, Michael Grant, Herne, Kent CT6 7LN (GB)
(74) Representative: Simpson, Alison Elizabeth Fraser

(57) **Abstract**

BPH in human males, unstable bladder contractions in human females, and LUTS in both males and females can be treated by orally administering an effective amount of a cGMP PDE inhibitor to a patient in need of such treatment. The cGMP PDE inhibitor can be co-administered with an α-adrenergic antagonist. When a combination of a cGMP PDE inhibitor and an α-adrenergic antagonist are administered, they can be administered together in a composition or administered separately. If administered separately, they can be embodied in a kit.

## Description

### Field Of The Invention

This invention relates to a method of treating benign prostatic hyperplasia (BPH) in men and also to treating unstable or irritable bladder contractions in women.

### Background Of The Invention

Benign Prostatic Hyperplasia (BPH), also called Benign Prostatic Hypertrophy, is a progressive, nearly universal condition in aging men characterized by a nodular enlargement of prostatic tissue resulting, through obstruction of the urethra, in variable degrees of bladder outlet obstruction. The disorder is not a major cause of death, but it is a leading cause of morbidity in elderly men and is associated with a variety of lower urinary tract symptoms (LUTS). LUTS in males include, *inter* alia, increased frequency of urination, nocturia, a poor urine stream and hesitancy or delay in starting the urine flow. Chronic consequences of BPH can include hypertrophy of bladder smooth muscle, a decompensated bladder and an increased incidence of urinary tract infection. The specific biochemical, histological and pharmacological properties of the prostate adenoma leading to the bladder outlet obstruction are not yet known. However, the development of BPH is considered to be an inescapable phenomenon for the aging male population. BPH is commonly seen in men over the age of 50, and is observed in approximately 70% of males over the age of 70. Currently, in the United States, the method of choice for treating BPH is the administration of α-adrenergic blockers and, to a lesser extent, surgery, usually involving transurethral resection of the prostate (TURP). The limitations of surgery for treating BPH include the morbidity associated with an operative procedure in elderly men, persistence or recurrence of obstructive and irritative symptoms, as well as the significant cost of surgery.

LUTS has become recognized as a separate condition which, although it was traditionally associated with BPH, is now known to have other etiologies as well, and is currently recognized as being associated with both men and women. It is noted that women, although they of course do not develop morphological BPH, nonetheless also suffer from LUTS due to unstable bladder contractions. Such unstable bladder contractions (and LUTS due to unstable bladder contractions) appears with greater frequency in the female population as the population ages, although unstable bladder contractions tend to appear in women at a younger age than BPH appears in men. Although LUTS in males and females share common features, the underlying pathophysiology may be different. LUTS in women manifests itself primarily in increased frequency of urination and poor control of voiding.

Agents which elevate cGMP levels are well known and can work through any of several mechanisms. Agents which selectively inhibit an enzyme predominantly involved in cGMP breakdown, for example a cGMP-dependent phosphodiesterase constitute one example. Other phosphodiesterases can also hydrolyze cGMP, and inhibitors of these enzymes including compounds such as rolipram, zaprinast and xanthine derivatives such as caffeine, theophylline and theobromine, can accordingly influence cGMP levels. Other compounds which increase cGMP levels can do so through different mechanisms including the activation of soluble guanylate cyclase or membrane-bound guanylate cyclase, either directly as in the case of atrial natriuretic peptide, or indirectly. Other compounds act to increase cellular cGMP levels by modulation of cytokines. Other classes of cGMP elevators include muscarinic agonists, which can elevate cGMP levels without altering phosphodiesterase activity. Some prostaglandins such as PGE₁ are also known cGMP elevators. Kanba et. al., J. Neurochem., Vol. 57, No. 6, 1991.

In particular, cyclic guanosine 3',5'-monophosphate phosphodiesterase (cGMP PDE) inhibitors are widely known as cardiovascular agents for the treatment of conditions such as angina, hypertension, and congestive heart failure. More recently cGMP PDE inhibitors capable of inhibiting type V phosphodiesterase (cGMP PDE_{V}) have been found to be effective for the treatment of impotence, importantly by oral administration. See, for example, PCT/EP94/01580, published as WO 94/28902 which designates, *inter alia,* the United States, and which is herein incorporated by reference.

### Summary Of The Invention

This invention provides a method for treating BPH in men and unstable bladder contractions in women, comprising administering, to a patient in need of such treatment, an effective amount of a cGMP elevator. It is noted that the treatment involves, *inter alia,* the remediation of lower urinary tract symptoms (LUTS) in both men and women, the LUTS being associated with BPH and with unstable bladder contractions, respectively.

The invention further provides a method for the treatment of LUTS in men and women, regardless of etiology. Thus if LUTS in a human male or human female is associated with an underlying condition other than BPH or unstable bladder contractions, such LUTS can be treated by means of this invention.

Thus a preferred embodiment is the use of a cGMP PDE_{V} elevator in the treatment of BPH in men.

Another preferred embodiment is the use of a cGMP PDE_{V} elevator in the treatment of unstable bladder contractions in women.

Another preferred embodiment is the use of a cGMP PDE_{V} elevator in the treatment of LUTS, regardless of etiology, in men and women.

Another preferred embodiment is the use of a cGMP PDE_{V} elevator in the treatment of LUTS associated with BPH in human males.

Another preferred embodiment is the use of a cGMP PDE_{V} elevator in the treatment of LUTS associated with unstable bladder contractions in human females.

Preferred as the cGMP elevator are cGMP PDE inhibitors. cGMP PDE inhibitors which are selective for cGMP PDEs rather than cyclic adenosine 3',5'-monophosphate phosphodiesterases (cAMP PDEs) and/or which are selective inhibitors of the cGMP PDE_{V} isoenzyme are particularly preferred. Such particularly preferred cGMP PDE inhibitors are disclosed in US patents 5,250,534, 5,346,901, 5,272,147, and in the international patent application published as WO 94/28902 designating, *inter alia,* the U. S., each of which is incorporated herein by reference. Additional preferred cGMP PDE inhibitors include those which can be taken "on demand", as opposed to needing to be taken chronically.

Preferred cGMP PDE_{V} inhibitors include compounds of formula (I): 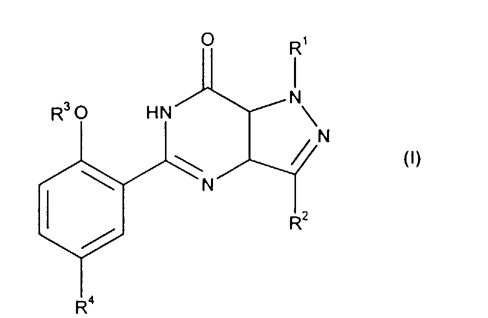 wherein:
R¹ is H; C₁-C₃ alkyl; C₁-C₃ perfluoroalkyl; or C₃-C₅ cycloalkyl;
R² is H; C₁-C₆ alkyl optionally substituted with C₃-C₆ cycloalkyl; C₁-C₃ perfluoroalkyl; or C₃-C₆ cycloalkyl;
R³ is C₁-C₆ alkyl optionally substituted with C₃-C₆ cycloalkyl; C₁-C₆ perfluoroalkyl; C₃-C₅ cycloalkyl; C₃-C₆ alkenyl; or C₃-C₆ alkynyl;
R⁴ is C₁-C₄ alkyl optionally substituted with OH, NR⁵R⁶, CN, CONR⁵R⁶ or CO₂R⁷; C₂-C₄ alkenyl optionally substituted with CN, CONR⁵R⁶ or CO₂R⁷; C₂-C₄ alkanoyl optionally substituted with NR⁵R⁶; (hydroxy)C₂-C₄ alkyl optionally substituted with NR⁵R⁶; (C₂-C₃ alkoxy)C₁-C₂ alkyl optionally substituted with OH or NR⁵R⁶; CONR⁵R⁶; CO₂R⁷; halo; NR⁵R⁶; NHSO₂NR⁵R⁶; NHSO₂R⁸; SO₂NR⁹R¹⁰; or phenyl, pyridyl, pyrimidinyl, imidazolyl, oxazolyl, thiazolyl, thienyl or triazolyl any of which is optionally substituted with methyl;
R⁵ and R⁶ are each independently H or C₁-C₄ alkyl, or together with the nitrogen atom to which they are attached form a pyrrolidinyl, piperidino, morpholino, 4-N(R¹¹)-piperazinyl or imidazolyl group wherein said group is optionally substituted with methyl or OH;
R⁷ is H or C₁-C₄ alkyl;
R⁸ is C₁-C₃ alkyl optionally substituted with NR⁵R⁶;
R⁹ and R¹⁰ together with the nitrogen atom to which they are attached form a pyrrolidinyl, piperidino, morpholino or 4-N(R¹²)-piperazinyl group wherein said group is optionally substituted with C₁-C₄ alkyl, C₁-C₃ alkoxy, NR¹³R¹⁴ or CONR¹³R¹⁴;
R¹¹ is H; C₁-C₃ alkyl optionally substituted with phenyl; (hydroxy)C₂-C₃ alkyl; or C₁-C₄ alkanoyl;
R¹² is H; C₁-C₆ alkyl; (C₁-C₃ alkoxy)C₂-C₆ alkyl; (hydroxy)C₂-C₆ alkyl; (R¹³R¹⁴N)C₂-C₆ alkyl; (R¹³R¹⁴NOC)C₁-C₆ alkyl; CONR¹³R¹⁴; CSNR¹³R¹⁴; or C(NH)NR¹³R¹⁴; and
R¹³ and R¹⁴ are each independently H; C₁-C₄ alkyl; (C₁-C₃ alkoxy)C₂-C₄ alkyl; or (hydroxy)C₂-C₄ alkyl;
or a pharmaceutically acceptable salt thereof;
or a pharmaceutically acceptable composition containing either entity.

Preferred cGMP PDE_{V} inhibitors include sildenafil which has the structure: 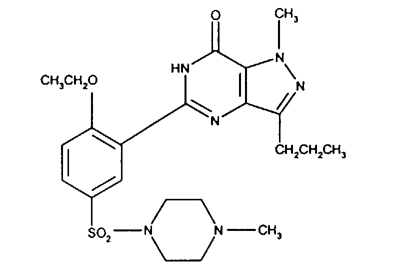 and pharmaceutically acceptable salts thereof, and the compound having the structure: 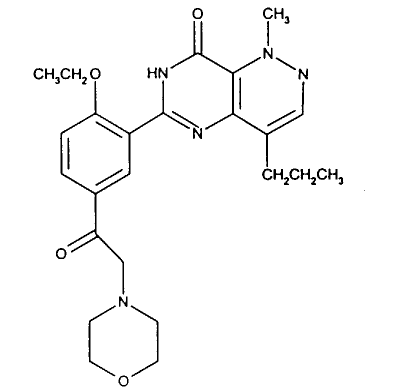 and pharmaceutically acceptable salts thereof. The second compound is disclosed, for example, in US patents 5,272,147 and 5,426,107, both of which are incorporated herein by reference.

A preferred pharmaceutically acceptable salt of sildenafil for use in this invention is the citrate salt, disclosed in co-pending U. S. Application No. 08/944,546 filed October 7, 1997 and incorporated herein by reference.

Also preferred as cGMP PDE_{V} inhibitors are compounds disclosed in PCT/EP95/00183, published as WO 95/19978 and which designates, *inter alia,* the United States, herein incorporated by reference, said compounds having the formula 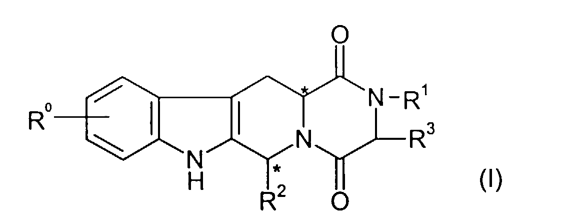 and salts and solvates thereof, in which:
R⁰ represents hydrogen, halogen or C₁-C₆alkyl,;
R¹ represents hydrogen, C₁-C₆alkyl, C₂-C₆alkenyl, C₂-C₆alkynyl, haloC₁-C₆alkyl, C₃-C₈cycloalkyl,
C₃-C₈cycloalkylC₁-C₃alkyl, arylC₁-C₃alkyl or heteroarylC₁-C₃alkyl;
R² represents an optionally substituted monocyclic aromatic ring selected from benzene, thiophene, furan and pyridine or an optionally substituted bicyclic ring 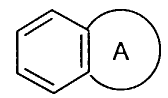 attached to the rest of the molecule via one of the benzene ring carbon atoms and wherein the fused ring A is a 5- or 6- membered ring which may be saturated or partially or fully unsaturated and comprises carbon atoms and optionally one or two heteroatoms selected from oxygen, sulphur and nitrogen; and
R³ represents hydrogen or C₁-C₃alkyl, or R¹ and R³ together represent a 3- or 4-membered alkyl or alkenyl chain.

A preferred subset of compounds having formula la (also disclosed in WO 95/19978) includes compounds of the formula 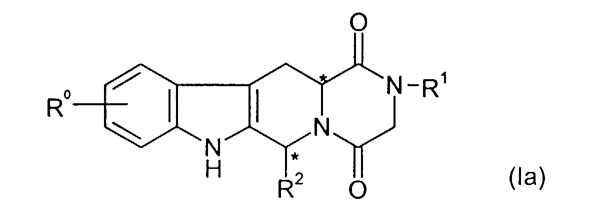 and salts and solvates thereof, in which:
R⁰ represents hydrogen, halogen or C₁-C₆alkyl;
R¹ represents hydrogen, C₁-C₆alkyl, haloCᵢ-C₆alkyl, C₃-C₈cycloalkyl, C₃-C₈cycloalkyl-C₁-C₃alkyl, arylC₁-C₃alkyl or heteroarylC₁-C₃alkyl; and
R² represents an optionally substituted monocyclic aromatic ring selected from benzene, thiophene, furan and pyridine or an optionally substituted bicyclic ring 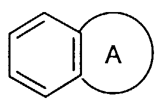 attached to the rest of the molecule via one of the benzene ring carbon atoms and wherein the fused ring A is a 5- or 6- membered ring which may be saturated or partially or fully unsaturated and comprises carbon atoms and optionally one or two heteroatoms selected from oxygen, sulphur and nitrogen.

A cGMP PDE inhibitor may be co-administered together with an α-adrenergic antagonist (also referred to herein as an "α-antagonist") to treat BPH, unstable bladder contractions and/or LUTS. "Co-administration" when used in this specification and the appendant claims, for example in referring to a combination of an α-adrenergic antagonist and a cGMP PDE inhibitor, means that the individual compounds can be administered together in a composition if the route of administration for each component is the same. "Co-administration" also includes administering a cGMP PDE inhibitor and an α-adrenergic antagonist separately but as part of the same therapeutic treatment program or regimen, and it is contemplated that separate administration of each compound, at different times and by different routes, will sometimes be recommended. Thus, the two compounds need not necessarily be administered at essentially the same time. It is possible and contemplated that the compounds may be administered at different times, even on different days, but as part of the same regimen.

Whether co-administered separately or together in a composition, it is most preferred that both compounds be administered in an oral dosage form.

The α-antagonist can be selective for either α₁- or α₂-adrenergic receptors (sometimes herein abbreviated as "adrenoceptor"), or it can be non selective, exhibiting antagonist activity at both α₁- and at α₂. Non selective antagonists are preferred. Antagonists selective for the α₁-adrenoceptor are more preferred.

Specific combinations of an α-antagonist and a cGMP elevator useful in this invention include any α-adrenoceptor antagonist in combination with sildenafil. Combinations of sildenafil, especially sildenafil citrate, with an α₁-selective adrenergic antagonist are preferred.

Preferred α-antagonists include doxazosin, terazosin, abanoquil, and prazosin, and the pharmaceutically acceptable salts thereof (especially doxazosin mesylate, terazosin hydrochloride, abanoquil mesylate and prazosin hydrochloride), which have been reported to be selective for α₁-adrenoceptors. Particularly preferred specific combinations for co-administration include any of these in combination with sildenafil or a pharmaceutically acceptable salt thereof, particularly the citrate salt. Most preferred are sildenafil citrate in combination with doxazosin mesylate or abanoquil mesylate.

Examples of additional α-antagonists include alfuzosin, indoramin, naftopidil, phentolamine, tamsulosin, trazodone, dapiprazole, phenoxybenzamine, idazoxan, efaroxan, and yohimbine, and also pharmaceutically acceptable salts thereof. Also useful are the rauwolfa alkaloids. Of these, phenoxybenzamine, phentolamine, trazodone, and dapiprazole are reported to be non-selective. Rauwolfa alkaloids, idazoxan, efaroxan and yohimbine are reported to be selective for α₂ receptors. The other specific compounds above are reported to be selective for α₁receptors.

Further α-antagonists which are well known in the art and are reported to be specific for α₁include:

Recordati 15/2739 which has the structure

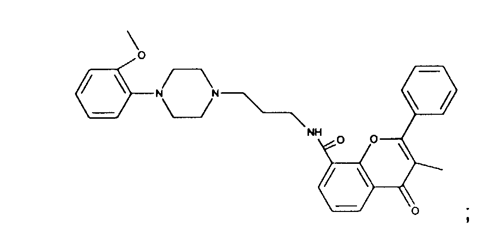

SNAP 1069 which has the structure

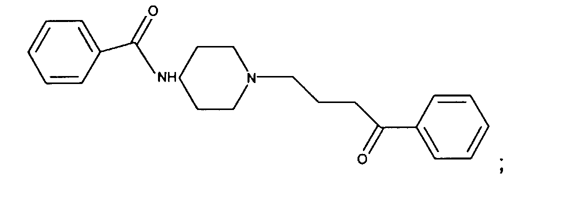

SNAP 5089 which has the structure

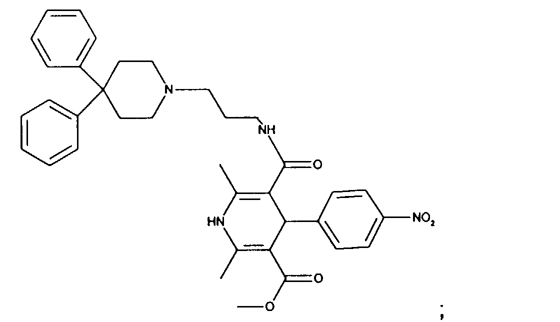

RS 17053 which has the structure 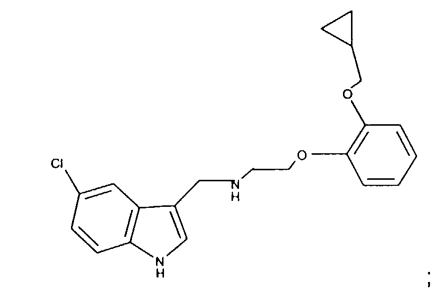 and

SL 89.0591 which has the structure

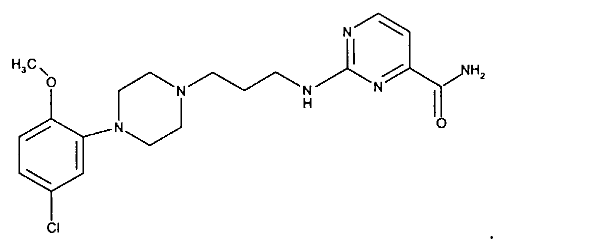

Since the present invention has an aspect that relates to the treatment of BPH, unstable bladder contractions, and/or LUTS by treatment with a combination of compounds which may be co-administered separately, the invention also relates to combining separate pharmaceutical compositions in kit form. The kit comprises two separate pharmaceutical compositions: (1) a composition comprising a compound selected from α-adrenergic receptor antagonists, plus a pharmaceutically acceptable carrier or diluent; and (2) a composition comprising a compound selected from agents which elevate cGMP levels, plus a pharmaceutically acceptable carrier or diluent. The amounts of (1) and (2) are such that, when co-administered separately, the condition of BPH or unstable bladder contractions and/or LUTS, is treated and/or remediated. The kit comprises a container for containing the separate compositions such as a divided bottle or a divided foil packet, wherein each compartment contains a plurality of dosage forms (e.g., tablets) comprising (1) or (2). Alternatively, rather than separating the active ingredient-containing dosage forms, the kit may contain separate compartments each of which contains a whole dosage which in tum comprises separate dosage forms. An example of this type of kit is a blister pack wherein each individual blister contains two (or more) tablets, one (or more) tablet(s) comprising pharmaceutical composition (1), and the second (or more) tablet(s) comprising pharmaceutical composition (2). Typically the kit comprises directions for the administration of the separate components. The kit form is particularly advantageous when the separate components are administered in different dosage forms (e.g., oral and parenteral), are administered at different dosage intervals, or when titration of the individual components of the combination is desired by the prescribing physician. In the case of the instant invention a kit therefore comprises
(1) a therapeutically effective amount of a composition comprising a compound selected from α-adrenergic receptor antagonists, plus a pharmaceutically acceptable carrier or diluent, in a first dosage form;
(2) a therapeutically effective amount of a composition comprising a compound selected from compounds which elevate cGMP levels, plus a pharmaceutically acceptable carrier or diluent, in a second dosage form; and
(3) a container for containing said first and second dosage forms.

An example of such a kit, as described above, is a so-called blister pack. Blister packs are well known in the packaging industry and are widely used for the packaging of pharmaceutical unit dosage forms such as tablets, capsules, and the like. Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably, the strength of the sheet is such that the tablets or capsules can be removed from the blister pack by manually applying pressure on the recesses whereby an opening is formed in the sheet at the place of the recess. Tablet(s) or capsule(s) can then be removed via said opening.

It may be desirable to provide a memory aid on the kit, e.g., in the form of numbers next to the tablets or capsules whereby the numbers correspond with the days of the regimen during which the tablets or capsules so specified should be ingested. Another example of such a memory aid is a calendar printed on the card, e.g., as follows "First Week, Monday, Tuesday, ...etc... Second Week, Monday, Tuesday,...", etc. Other variations of memory aids will be readily apparent. A "daily dose" can be a single tablet or capsule or several pills or capsules to be taken on a given day. Also a daily dose of the first compound can consist of one tablet or capsule while a daily dose of the second compound can consist of several tablets or capsules and vice versa. The memory aid should reflect this.

### Detailed Description

The cGMP PDE inhibitors useful in this invention as cGMP elevators may be chosen from among any of those already known to the art or subsequently discovered and/or hereafter developed. Suitable cGMP PDE inhibitors include those disclosed in any of the following US patents, all of which are incorporated herein by reference:
a 5-substituted pyrazolo[4,3-d]pyrimidine-7-one as disclosed in US 4,666,908;
a griseolic acid derivative as disclosed in any of US 4,634,706, 4,783,532, 5,498,819, 5,532,369, 5,556,975, and 5,616,600;
a 2-phenylpurinone derivative as disclosed in US 4,885,301;
a phenylpyridone derivative as disclosed in US 5,254,571;
a fused pyrimidine derivative as disclosed in US 5,047,404;
a condensed pyrimidine derivative as disclosed in US 5,075,310;
a pyrimidopyrimidine derivative as disclosed in US 5,162,316;
a purine compound as disclosed in US 5,073,559;
a quinazoline derivative as disclosed in US 5,147,875;
a phenylpyrimidone derivative as disclosed in US 5,118,686;
an imidazoquinoxalinone derivative or its aza analog as disclosed in US 5,055,465 and 5,166,344;
a phenylpyrimidone derivative as disclosed in US 5,290,933;
a 4-aminoquinazoline derivative as disclosed in US 5,436,233 or 5,439,895;
a 4,5-dihydro-4-oxo-pyrrolo[1,2-a]quinoxaline derivative as disclosed in US 5,405,847;
a polycyclic guanine derivative as disclosed in US 5,393,755;
a nitogenous heterocyclic compound as disclosed in US 5,576,322;
a quinazoline derivative as disclosed in US 4,060,615;
a 6-heterocyclyl pyrazolo[3,4-d]pyrimidin-4-one as disclosed in US 5,294,612; and
a 4-aminoquinazoline derivative as disclosed in US patent 5436233;

Other disclosures of cGMP PDE inhibitors include the following, all of which are herein incorporated by reference:
European patent Application (EPA) publication no. 0428268;
European patent 0442204;
International patent application publication no. WO 94/19351;
Japanese patent application 5-222000;
European Journal Of Pharmacology, 251, (1994), 1;
International patent application publication no. WO 94/22855;
a pyrazolopyrimidine derivative as disclosed in European patent application 0636626;
a 4-aminopyrimidine derivative as disclosed in European patent application 0640599;
an imidazoquinazoline derivative as disclosed in International patent application W095/06648;
an anthranilic acid derivative as disclosed in International patent application WO95/18097;
a tetracyclic derivative as disclosed in International patent application WO95/19978;
an imidazoquinazoline derivative as disclosed in Euripean patent application 0668280; and
a quinazoline compound as disclosed in Euripean patent application 0669324.

The cGMP PDE inhibition of a compound can be determined by standard assays known to the art, for example as disclosed in US 5,250,534, incorporated herein by reference. Compounds which are selective inhibitors of cGMP PDE relative to cAMP PDE are preferred, and determination of such compounds is also taught in US 5,250,534. Particularly preferred are compounds which selectively inhibit the PDE_{V} isoenzyme, as disclosed in the aforementioned PCT/EP94/01580, published as WO 94/28902.

α-Antagonists and salts thereof, in addition to those specifically identified above, have been widely disclosed in the patent literature, including U.S. patents 4,188,390, 4,026,894, 3,511,836, 4,315,007, 3,527,761, 3,997,666, 2,503,059, 4,703,063, 3,381,009, 4,252,721, and 2,599,000, each of which is incorporated herein by reference.

The α-antagonism of a compound, and therefore its suitability for use in the present invention, can be determined using a number of conventional assays *in vitro.* Suitable assays include those disclosed in U. S. patent 5,599,810 which employ rabbit aorta to determine α₁-adrenoceptor antagonist activity and guinea pig left atrium to determine α₂-adrenergic antagonist activity, and in U.S. 5,340,814 which employ rat brain cortex membranes to determine both α₁and α₂ antagonist activity. Both of those patents are incorporated herein by reference.

If a combination of a cGMP PDE elevator is used, for example a cGMP PDE inhibitor, or a composition comprising both a cGMP PDE elevator and an α-antagonist, its activity in treating BPH/unstable bladder contractions/LUTS can be demonstrated by *in vivo* tests as known in the art. For example, a rat model may be employed as disclosed in U.S. patent 5,726,202 (see example 1 therein). An anesthetized dog model may also be employed as disclosed in U.S. patent 4,755,507. Both patents are incorporated herein by reference. Suitability for treatment of unstable bladder contractions in women (and the LUTS which are associated therewith) can also be shown by the same tests.

The routes of administration for a cGMP elevator if administered alone, or for co-administering a cGMP elevator plus an α-adrenergic antagonist whether separately or together in a composition, can be any of those known to the art such as oral, parenteral via by intravenous injection, by injection via subcutaneous or intramuscular depot, or transdermal.

Oral administration is preferred. Each component can be formulated as known in the art, usually together with a pharmaceutically acceptable carrier or diluent, for example as a tablet, capsule, lozenge, troche, elixir, solution, or suspension for oral administration, or in a suitable depot vehicle for parenteral administration. In a preferred embodiment, the cGMP elevator and the α-antagonist are each co-administered orally, together or separately.

The exact dose of each component administered will, of course, differ depending on the specific components prescribed, on the subject being created, on the severity of the LUTS being treated, on the manner of administration and on the judgment of the prescribing physician. Thus, because of patient-to-patient variability, the dosages given below are a guideline and the physician may adjust doses of the compounds to achieve the treatment that the physician considers appropriate for the patient, male or female. In considering the degree of treatment desired, the physician must balance a variety of factors such as the age of the patient and the presence of other diseases or conditions (e.g., cardiovascular disease). In general, for the treatment of BPH, unstable bladder contractions, and LUTS the cGMP elevator will be administered in men and women in a range of from 0.5 to 200 mg per day, preferably 2.5 to 125 mg per day, more preferably 10-100 mg per day. The α-adrenergic antagonist, when co-administered, will generally be administered in an amount of from 0.01 mg to 50 mg per day, preferably from 0.5 to 10 mg per day. Generally, if the route of administration is by injection (e.g., intravenous), the injected amount is in a volume which usually will not exceed 1 ml. The injectable carrier or diluent is typically sterile physiological saline or another physiologically acceptable salt solution. Oral administration of prostaglandins is also feasible. Japanese Journal of Urology, 83(10):1655-1661, (1992).

As previously disclosed, the combination of cGMP elevator and α-adrenergic antagonist can be administered as a composition. Thus, the compounds of this invention can be administered together in any conventional oral, parenteral, or transdermal dosage form, usually also together with a pharmaceutically acceptable carrier or diluent.

For oral administration a pharmaceutical composition can take the form of solutions, suspensions, tablets, pills, capsules, powders, and the like. Tablets containing various excipients such as sodium citrate, calcium carbonate and calcium phosphate are employed along with various disintegrants such as starch and preferably potato or tapioca starch and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, the compounds of this invention can be combined with various sweetening agents, flavoring agents, coloring agents, emulsifying agents and/or suspending agents, as well as such diluents as water, ethanol, propylene glycol, glycerin and various like combinations thereof.

For purposes of parenteral administration, solutions in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes. In this connection, the sterile aqueous media employed are all readily obtainable by standard techniques well-known to those skilled in the art.

For purposes of transdermal administration, dilute sterile, aqueous or partially aqueous solutions (usually in about 0.1% to 5% concentration), otherwise similar to the above parenteral solutions, are prepared.

Methods of preparing various pharmaceutical compositions with a certain amount of active ingredient are known, or will be apparent in light of this disclosure, to those skilled in this art. For examples of methods of preparing pharmaceutical compositions, see Remington's Pharmaceutical Sciences, Mack Publishing Company, Easter, Pa., 15th Edition (1975).

## Claims

1. A method for treating BPH comprising administering, to a male patient in need of such treatment, an effective amount of a cGMP elevator.

2. A method as defined in claim 1, wherein said cGMP elevator is a cGMP PDE_{V} inhibitor.

3. A method as defined in claim 2, wherein said cGMP PDE_{V} inhibitor has formula (I): 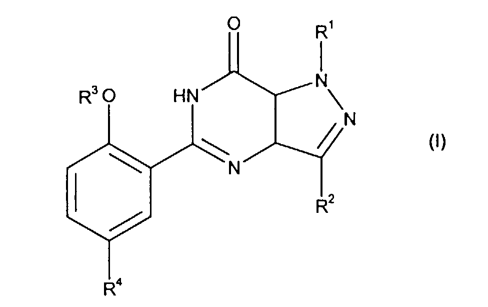 wherein:
R¹ is H; C₁-C₃ alkyl; C₁-C₃ perfluoroalkyl; or C₃-C₅ cycloalkyl;
R² is H; C₁-C₆ alkyl optionally substituted with C₃-C₆ cycloalkyl; C₁-C₃ perfluoroalkyl; or C₃-C₆ cycloalkyl;
R³ is C₁-C₆ alkyl optionally substituted with C₃-C₆ cycloalkyl; C₁-C₆ perfluoroalkyl; C₃-C₅ cycloalkyl; C₃-C₆ alkenyl; or C₃-C₆ alkynyl;
R⁴ is C₁-C₄ alkyl optionally substituted with OH, NR⁵R⁶, CN, CONR⁵R⁶ or CO₂R⁷; C₂-C₄ alkenyl optionally substituted with CN, CONR⁵R⁶ or CO₂R⁷; C₂-C₄ alkanoyl optionally substituted with NR⁵R⁶; (hydroxy)C₂-C₄ alkyl optionally substituted with NR⁵R⁶; (C₂-C₃ alkoxy)C₁-C₂ alkyl optionally substituted with OH or NR⁵R⁶; CONR⁵R⁶; CO₂R⁷; halo; NR⁵R⁶; NHSO₂NR⁵R⁶; NHSO₂R⁸; SO₂NR⁹R¹⁰; or phenyl, pyridyl, pyrimidinyl, imidazolyl, oxazolyl, thiazolyl, thienyl or triazolyl any of which is optionally substituted with methyl;
R⁵ and R⁶ are each independently H or C₁-C₄ alkyl, or together with the nitrogen atom to which they are attached form a pyrrolidinyl, piperidino, morpholino, 4-N(R¹¹)-piperazinyl or imidazolyl group wherein said group is optionally substituted with methyl or OH;
R⁷ is H or C₁-C₄ alkyl;
R⁸ is C₁-C₃ alkyl optionally substituted with NR⁵R⁶;
R⁹ and R¹⁰ together with the nitrogen atom to which they are attached form a pyrrolidinyl, piperidino, morpholino or 4-N(R¹²)-piperazinyl group wherein said group is optionally substituted with C₁-C₄ alkyl, C₁-C₃ alkoxy, NR¹³R¹⁴ or CONR¹³R¹⁴;
R¹¹ is H; C₁-C₃ alkyl optionally substituted with phenyl; (hydroxy)C₂-C₃ alkyl; or C₁-C₄ alkanoyl;
R¹² is H; C₁-C₆ alkyl; (C₁-C₃ alkoxy)C₂-C₆ alkyl; (hydroxy)C2-C6 alkyl; (R¹³R¹⁴N)C₂-C₆ alkyl; (R¹³R¹⁴NOC)C₁-C₆ alkyl; CONR¹³R¹⁴; CSNR¹³R¹⁴; or C(NH)NR¹³R¹⁴; and
R¹³ and R¹⁴ are each independently H; C₁-C₄ alkyl; (C₁-C₃ alkoxy)C₂-C₄ alkyl; or (hydroxy)C₂-C₄ alkyl;
or is a pharmaceutically acceptable salt thereof;
or is a pharmaceutically acceptable composition containing either entity.

4. A method as defined in claim 3, wherein said compound is sildenafil or a pharmaceutically acceptable salt thereof.

5. A method as defined in claim 4, wherein said salt is the citrate.

6. A method as defined in claim 3, wherein said compound has the structure: 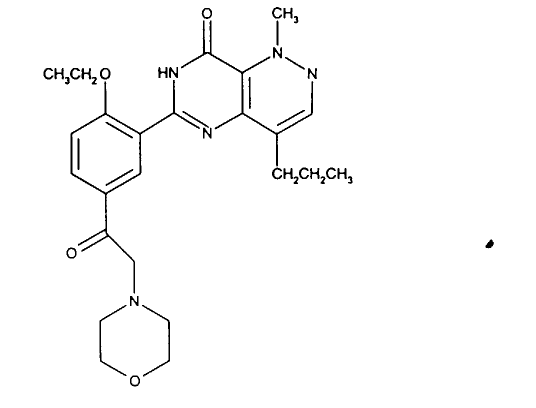 or is a pharmaceutically acceptable salt thereof.

7. A method as defined in claim 2, wherein said cGMP PDE_{V} inhibitor has the structure 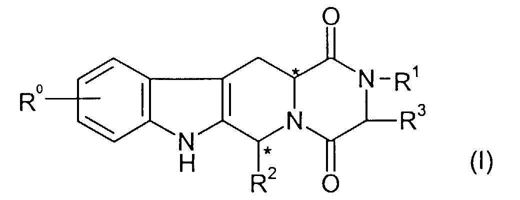 and salts and solvates thereof, in which:
R⁰ represents hydrogen, halogen or C₁-C₆alkyl,;
R¹ represents hydrogen, C₁-C₆alkyl, C₂-C₆alkenyl, C₂-C₆alkynyl, haloC₁-C₆alkyl, C₃-C₈cycloalkyl,
C₃-C₈cycloalkylC₁-C₃alkyl, arylC₁-C₃alkyl or heteroarylC₁-C₃alkyl;
R² represents an optionally substituted monocyclic aromatic ring selected from benzene, thiophene, furan and pyridine or an optionally substituted bicyclic ring 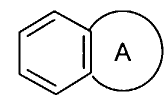 attached to the rest of the molecule via one of the benzene ring carbon atoms and wherein the fused ring A is a 5- or 6- membered ring which may be saturated or partially or fully unsaturated and comprises carbon atoms and optionally one or two heteroatoms selected from oxygen, sulphur and nitrogen; and
R³ represents hydrogen or C₁-C₃alkyl, or R¹ and R³ together represent a 3- or 4- membered alkyl or alkenyl chain.

8. A method as defined in claim 1, further comprising co-administering, in addition to said cGMP elevator, an α-adrenergic antagonist.

9. A method as defined in claim 8, wherein said cGMP elevator is a cGMP PDE_{V} inhibitor.

10. A method as defined in claim 9, wherein said cGMP PDE_{V} inhibitor and said α-adrenergic antagonist are embodied as a kit.

11. A method for treating unstable bladder contractions in women, comprising administering, to a female patient in need of such treatment, an effective amount of a cGMP elevator.

12. A method as defined in claim 11, wherein said cGMP elevator is a cGMP PDE_{V} inhibitor.

13. A method as defined in claim 12, wherein said cGMP PDE_{V} inhibitor has formula (I): 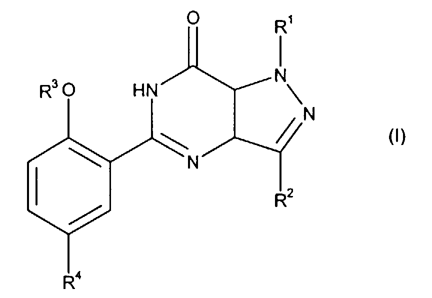 wherein:
R¹ is H; C₁-C₃ alkyl; C₁-C₃ perfluoroalkyl; or C₃-C₅ cycloalkyl;
R² is H; C₁-C₆ alkyl optionally substituted with C₃-C₆ cycloalkyl; C₁-C₃ perfluoroalkyl; or C₃-C₆ cycloalkyl;
R³ is C₁-C₆ alkyl optionally substituted with C₃-C₆ cycloalkyl; C₁-C₆ perfluoroalkyl; C₃-C₅ cycloalkyl; C₃-C₆ alkenyl; or C₃-C₆ alkynyl;
R⁴ is C₁-C₄ alkyl optionally substituted with OH, NR⁵R⁶, CN, CONR⁵R⁶ or CO₂R⁷; C₂-C₄ alkenyl optionally substituted with CN, CONR⁵R⁶ or CO₂R⁷; C₂-C₄ alkanoyl optionally substituted with NR⁵R⁶; (hydroxy)C2-C4 alkyl optionally substituted with NR⁵R⁶; (C₂-C₃ alkoxy)C₁-C₂ alkyl optionally substituted with OH or NR⁵R⁶; CONR⁵R⁶; CO₂R⁷; halo; NR⁵R⁶; NHSO₂NR⁵R⁶; NHSO₂R⁸; SO₂NR⁹R¹⁰; or phenyl, pyridyl, pyrimidinyl, imidazolyl, oxazolyl, thiazolyl, thienyl or triazolyl any of which is optionally substituted with methyl;
R⁵ and R⁶ are each independently H or C₁-C₄ alkyl, or together with the nitrogen atom to which they are attached form a pyrrolidinyl, piperidino, morpholino, 4-N(R¹¹)-piperazinyl or imidazolyl group wherein said group is optionally substituted with methyl or OH;
R⁷ is H or C₁-C₄ alkyl;
R⁸ is C₁-C₃ alkyl optionally substituted with NR⁵R⁶;
R⁹ and R¹⁰ together with the nitrogen atom to which they are attached form a pyrrolidinyl, piperidino, morpholino or 4-N(R¹²)-piperazinyl group wherein said group is optionally substituted with C₁-C₄ alkyl, C₁-C₃ alkoxy, NR¹³R¹⁴ or CONR¹³R¹⁴;
R¹¹ is H; C₁-C₃ alkyl optionally substituted with phenyl; (hydroxy)C₂-C₃ alkyl; or C₁-C₄ alkanoyl;
R¹² is H; C₁-C₆ alkyl; (C₁-C₃ alkoxy)C₂-C₆ alkyl; (hydroxy)C₂-C₆ alkyl; (R¹³R¹⁴N)C₂-C₆ alkyl; (R¹³R¹⁴NOC)C₁-C₆ alkyl; CONR¹³R¹⁴; CSNR¹³R¹⁴; or C(NH)NR¹³R¹⁴; and
R¹³ and R¹⁴ are each independently H; C₁-C₄ alkyl; (C₁-C₃ alkoxy)C₂-C₄ alkyl; or (hydroxy)C₂-C₄ alkyl;
or is a pharmaceutically acceptable salt thereof;
or is a pharmaceutically acceptable composition containing either entity.

14. A method as defined in claim 13, wherein said compound is sildenafil or a pharmaceutically acceptable salt thereof.

15. A method as defined in claim 14, wherein said salt is the citrate.

16. A method as defined in claim 13, wherein said compound has the structure: 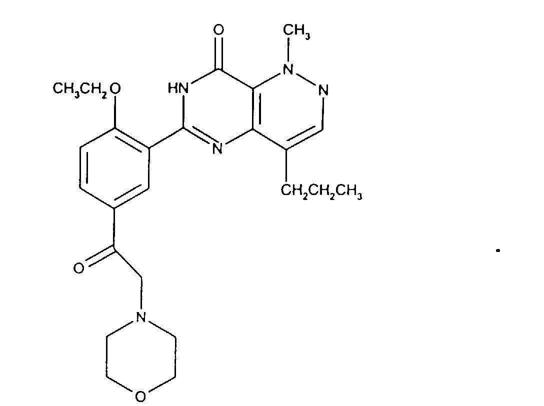 or is a pharmaceutically acceptable salt thereof.

17. A method as defined in claim 12, wherein said cGMP PDE_{V} inhibitor has the structure 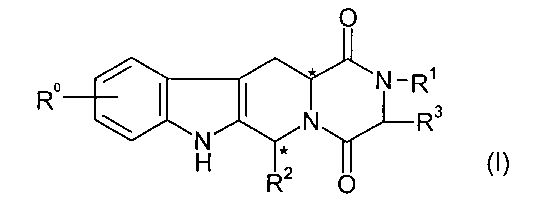 and salts and solvates thereof, in which:
R⁰ represents hydrogen, halogen or C₁-C₆alkyl,;
R¹ represents hydrogen, C₁-C₆alkyl, C₂-C₆alkenyl, C₂-C₆alkynyl, haloC₁-C₆alkyl, C₃-C₈cycloalkyl,
C₃-C₈cycloalkylC₁-C₃alkyl, arylC₁-C₃alkyl or heteroarylC₁-C₃alkyl;
R² represents an optionally substituted monocyclic aromatic ring selected from benzene, thiophene, furan and pyridine or an optionally substituted bicyclic ring 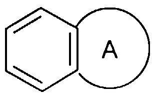 attached to the rest of the molecule via one of the benzene ring carbon atoms and wherein the fused ring A is a 5- or 6- membered ring which may be saturated or partially or fully unsaturated and comprises carbon atoms and optionally one or two heteroatoms selected from oxygen, sulphur and nitrogen; and
R³ represents hydrogen or C₁-C₃alkyl, or R¹ and R³ together represent a 3- or 4- membered alkyl or alkenyl chain.

18. A method as defined in claim 11, further comprising co-administering, in addition to said cGMP elevator, an α-adrenergic antagonist.

19. A method as defined in claim 18, wherein said cGMP elevator is a cGMP PDE_{V} inhibitor.

20. A method as defined in claim 19, wherein said cGMP PDE_{V} inhibitor and said α-adrenergic antagonist are embodied as a kit.

21. A method for treating lower urinary tract symptoms (LUTS), comprising administering, to a patient in need of such treatment, an effective amount of a cGMP elevator.

22. A method as defined in claim 21, wherein said cGMP elevator is a cGMP PDE_{V} inhibitor.

23. A method as defined in claim 22, wherein said cGMP PDE_{V} inhibitor has formula (I): 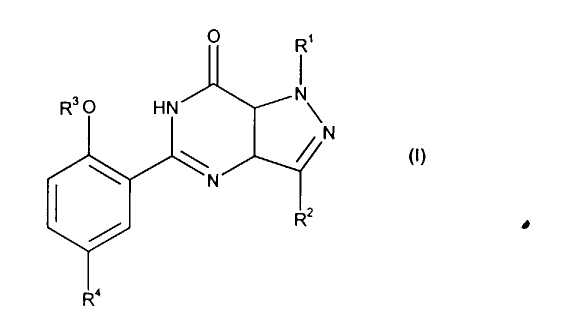 wherein:
R¹ is H; C₁-C₃ alkyl; C₁-C₃ perfluoroalkyl; or C₃-C₅ cycloalkyl;
R² is H; C₁-C₆ alkyl optionally substituted with C₃-C₆ cycloalkyl; C₁-C₃ perfluoroalkyl; or C₃-C₆ cycloalkyl;
R³ is C₁-C₆ alkyl optionally substituted with C₃-C₆ cycloalkyl; C₁-C₆ perfluoroalkyl; C₃-C₅ cycloalkyl; C₃-C₆ alkenyl; or C₃-C₆ alkynyl;
R⁴ is C₁-C₄ alkyl optionally substituted with OH, NR⁵R⁶, CN, CONR⁵R⁶ or CO₂R⁷; C₂-C₄ alkenyl optionally substituted with CN, CONR⁵R⁶ or CO₂R⁷; C₂-C₄ alkanoyl optionally substituted with NR⁵R⁶; (hydroxy)C₂-C₄ alkyl optionally substituted with NR⁵R⁶; (C₂-C₃ alkoxy)C₁-C₂ alkyl optionally substituted with OH or NR⁵R⁶; CONR⁵R⁶; CO₂R⁷; halo; NR⁵R⁶; NHSO₂NR⁵R⁶; NHSO₂R⁸; SO₂NR⁹R¹⁰; or phenyl, pyridyl, pyrimidinyl, imidazolyl, oxazolyl, thiazolyl, thienyl or triazolyl any of which is optionally substituted with methyl;
R⁵ and R⁶ are each independently H or C₁-C₄ alkyl, or together with the nitrogen atom to which they are attached form a pyrrolidinyl, piperidino, morpholino, 4-N(R¹¹)-piperazinyl or imidazolyl group wherein said group is optionally substituted with methyl or OH;
R⁷ is H or C₁-C₄ alkyl;
R⁸ is C₁-C₃ alkyl optionally substituted with NR⁵R⁶;
R⁹ and R¹⁰ together with the nitrogen atom to which they are attached form a pyrrolidinyl, piperidino, morpholino or 4-N(R¹²)-piperazinyl group wherein said group is optionally substituted with C₁-C₄ alkyl, C₁-C₃ alkoxy, NR¹³R¹⁴ or CONR¹³R¹⁴;
R¹¹ is H; C₁-C₃ alkyl optionally substituted with phenyl; (hydroxy)C₂-C₃ alkyl; or C₁-C₄ alkanoyl;
R¹² is H; C₁-C₆ alkyl; (C₁-C₃ alkoxy)C₂-C₆ alkyl; (hydroxy)C₂-C₆ alkyl; (R¹³R¹⁴N)C₂-C₆ alkyl; (R¹³R¹⁴NOC)C₁-C₆ alkyl; CONR¹³R¹⁴; CSNR¹³R¹⁴; or C(NH)NR¹³R¹⁴; and
R¹³ and R¹⁴ are each independently H; C₁-C₄ alkyl; (C₁-C₃ alkoxy)C₂-C₄ alkyl; or (hydroxy)C₂-C₄ alkyl;
or is a pharmaceutically acceptable salt thereof;
or is a pharmaceutically acceptable composition containing either entity.

24. A method as defined in claim 23, wherein said compound is sildenafil or a pharmaceutically acceptable salt thereof.

25. A method as defined in claim 24, wherein said salt is the citrate.

26. A method as defined in claim 23, wherein said compound has the structure: 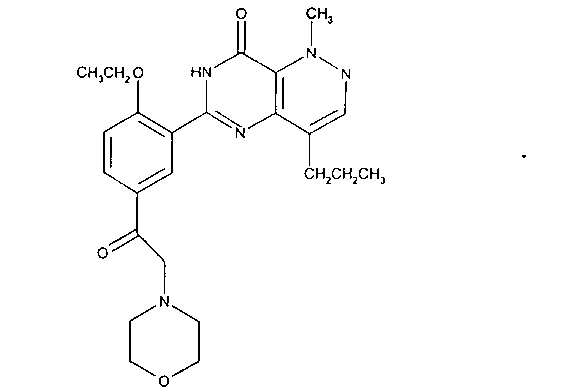 or is a pharmaceutically acceptable salt thereof.

27. A method as defined in claim 22, wherein said cGMP PDE_{V} inhibitor has the structure 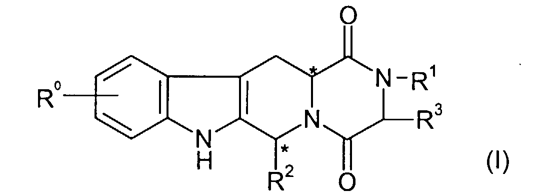 and salts and solvates thereof, in which:
R⁰ represents hydrogen, halogen or C₁-C₆alkyl,;
R¹ represents hydrogen, C₁-C₆alkyl, C₂-C₆alkenyl, C₂-C₆alkynyl, haloC₁-C₆alkyl, C₃-C₈cycloalkyl,
C₃-C₈cycloalkylC₁-C3alkyl, arylC₁-C₃alkyl or heteroarylC₁-C₃alkyl;
R² represents an optionally substituted monocyclic aromatic ring selected from benzene, thiophene, furan and pyridine or an optionally substituted bicyclic ring 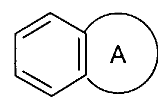 attached to the rest of the molecule via one of the benzene ring carbon atoms and wherein the fused ring A is a 5- or 6- membered ring which may be saturated or partially or fully unsaturated and comprises carbon atoms and optionally one or two heteroatoms selected from oxygen, sulphur and nitrogen; and
R³ represents hydrogen or C₁-C₃alkyl, or R¹ and R³ together represent a 3- or 4- membered alkyl or alkenyl chain.

28. A method as defined in claim 21, further comprising co-administering, in addition to said cGMP elevator, an α-adrenergic antagonist.

29. A method as defined in claim 28, wherein said cGMP elevator is a cGMP PDE_{V} inhibitor.

30. A method as defined in claim 29, wherein said cGMP PDE_{V} inhibitor and said α-adrenergic antagonist are embodied as a kit.
